(19) **Europäisches Patentamt / European Patent Office / Office européen des brevets**

(11) **EP 2 692 276 B1**

(12) # EUROPEAN PATENT SPECIFICATION

(45) Date of publication and mention
of the grant of the patent:
**27.09.2017 Bulletin 2017/39**

(51) Int Cl.:
*A61B 1/00* *(2006.01)*      *A61B 1/04* *(2006.01)*
*A61B 1/06* *(2006.01)*      *H04N 7/18* *(2006.01)*

(21) Application number: **12833097.4**

(22) Date of filing: **18.04.2012**

(86) International application number:
**PCT/JP2012/060445**

(87) International publication number:
**WO 2013/042396 (28.03.2013 Gazette 2013/13)**

(54) **MEDICAL INSTRUMENT**

MEDIZINISCHES INSTRUMENT

INSTRUMENT MÉDICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.09.2011 JP 2011207719**

(43) Date of publication of application:
**05.02.2014 Bulletin 2014/06**

(73) Proprietor: **Olympus Corporation
Tokyo 192-8507 (JP)**

(72) Inventors:
• **YAMAZAKI, Kenji
Hachioji-shi,
Tokyo 192-8507 (JP)**

• **IGARASHI, Makoto
Hachioji-shi,
Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(56) References cited:
**WO-A1-2007/116663**      **WO-A1-2010/044432**
**JP-A- 2006 122 502**      **JP-A- 2006 341 078**
**JP-A- 2010 069 063**      **US-A1- 2003 001 104**
**US-A1- 2006 082 647**      **US-A1- 2009 036 741**
**US-A1- 2010 265 321**      **US-A1- 2010 331 624**

**Description**

Technical Field

The present invention relates to a medical instrument, and particularly to a medical instrument that processes an image signal of returning light of light that is irradiated onto living tissue.

Background Art

Medical instruments that pick up an image of returning light of light that is irradiated onto living tissue and generate and output image signals are already in widespread use. For example, an endoscope apparatus is a medical instrument that includes an insertion portion that is inserted into a subject to acquire an image of living tissue that is obtained by picking up an image by means of an image pickup device provided at a distal end of the insertion portion, and that displays the obtained image on a monitor for use in diagnosis and the like.

Some endoscope apparatuses include not only a white-color-light observation mode that irradiates white color light onto living tissue and observes an image of reflected light from the living tissue, that is, a normal-light observation mode, but also include a special-light observation mode that irradiates illuminating light of a predetermined wavelength band onto living tissue and observes an image of returning light from the living tissue.

A narrow band observation mode as one special-light observation mode, for example, is a mode for observing a blood vessel image or a microstructure of a mucous membrane with good contrast. Another special-light observation mode is a fluorescence observation mode that irradiates narrow-band excitation light onto living tissue, and picks up an image of fluorescence emitted by a fluorescent substance in the living tissue.

In some cases an object other than living tissue is included in an image obtained in a special-light observation mode. For example, residue (residual stool, intestinal juice, bile or the like) may be mentioned as an object other living tissue.

Since residue or the like included in an image obtained by special-light observation is an interference factor when a surgeon diagnoses a lesion part, Japanese Patent Application Laid-Open Publication No. 2004-8230 proposes technology that, with respect to fluorescence observation, performs processing that identifies a region of an interference factor such as blood or residue that has adhered to living tissue. Further, Japanese Patent Application Laid-Open Publication No. 2003-79568 proposes technology that, with respect to fluorescence observation, performs processing that makes an interference region a different color to other regions so that a region of an interference factor such as blood or residue that is adhered to living tissue is not mistakenly identified as diseased tissue. In addition, Japanese Patent Application Laid-Open Publication No. 2007-125245 proposes technology that, with respect to fluorescence observation, changes a display form of a fluorescence image portion that results from residue so that the residue is identifiable on the fluorescence image.

However, under the special-light observation, for example, in the conventional narrow band observation mode for observing a blood vessel image or a microstructure of a mucous membrane with good contrast, there is a case where a yellow substance other than living tissue, for example, residue is displayed in a deep red color. This is because, when a predetermined color conversion is performed, as a result of the color conversion, a yellow color of an object viewed with the naked eye or in a white-color-light observation mode is converted into red.

However, since the red color is same as the color of blood, a surgeon or the like falsely recognizes for one moment with conditioned response that the object of red color in the image displayed on the monitor is blood, or the surgeon feels a sense of incongruity with the image. Since there is also a case where a subject to be examined using an endoscope views the monitor together with the surgeon, the subject to be examined is likely to have a similar false recognition against the object of red color.

In addition, when a blue pigment is sprayed on living tissue, the sprayed blue pigment is reproduced in a color such as green color or blue-green color on a narrow-band image.

In the narrow band observation mode, however, relatively thick blood vessels running in a deep part of mucous membrane are also reproduced in a color such as green color or blue-green color. The surgeon or the like sometimes feels a sense of incongruity, when an object of a color similar to the color of the blood vessels exists in an image.

The reason why the person who views the image feels a sense of incongruity with the image is that color tones of objects other than living tissue in the image displayed under the special-light observation mode are different from the color tone of the objects shown in the white-color-light mode.

Also in the techniques related to the above-described respective proposals, residue or the like is shown in a different color. However, there is no disclosure regarding a technique for generating an image to be displayed on a monitor as an image which does not give a sense of incongruity to a surgeon or the like under the special-light observation image mode.

US 2003/001104 A1 discloses a method and apparatus for obtaining fluorescence images by projecting an illuminating light containing excitation light onto a target subject and obtaining a diagnostic fluorescence image of the target subject based on the fluorescence emitted from the target subject upon the irradiation thereof by the excitation light, and a program for causing a computer to execute the fluorescence image obtaining method. White light and excitation light are projected onto a target subject to obtain respective standard and fluorescence images thereof. Because the color of the obstructing regions is different from that of either normal or diseased tissue, the color data of the standard image is computed, and the obstructing regions detected by determining whether or not the color data is outside a predetermined range. The fluorescence image is subjected to an exceptional display process of rendering the color of the obstructing regions a different color than that of the other regions, and the processed fluorescence diagnostic image is displayed on a monitor.

The present invention has been achieved in view of the above-described points and an object of the present invention is to provide a medical instrument that displays, under a special-light observation image mode, an object other than living tissue in a color tone that is similar to a color thereof in an image displayed in a white-color-light observation mode.

Disclosure of Invention

Means for Solving the Problem

A medical instrument according to one aspect of the present invention includes the features of claim 1.

Brief Description of the Drawings

Fig. 1 is a view that illustrates a configuration of an endoscope apparatus 1 according to a first embodiment of the present invention;
Fig. 2 is a view that illustrates an example of spectral characteristics of a narrow-band filter 25 according to the first embodiment of the present invention;
Fig. 3 is a view that illustrates a configuration of a table TBL that stores hue regions that are discriminated by a color discrimination circuit 47 as well as a discrimination reference thereof according to the first embodiment of the present invention;
Fig. 4 is a view for describing color spaces that are discriminated by the color discrimination circuit 47 as well as color correction processing thereof according to the first embodiment of the present invention;
Fig. 5 is a flowchart illustrating processing for color discrimination and color correction that is performed for each pixel by the color discrimination circuit 47 and a color correction circuit 48 according to the first embodiment of the present invention;
Fig. 6 is a view that illustrates an example of spectral characteristics of a narrow-band filter 25A and reflectance of objects according to a second embodiment of the present invention;
Fig. 7 is a view for describing color spaces that are discriminated by the color discrimination circuit 47 as well as color correction processing thereof according to the second embodiment of the present invention;
Fig. 8 is a view for describing differences in intensity with respect to residue and hemoglobin Hb, respectively, in signals Rin, Gin and Bin that are inputted to the color discrimination circuit 47 according to the second embodiment of the present invention;
Fig. 9 is a view that illustrates an example of spectral characteristics of the narrow-band filter 25A and reflectance of objects according to a Modification 2-1 of the second embodiment of the present invention;
Fig. 10 is a view for describing differences in intensity with respect to residue and hemoglobin Hb, respectively, in signals Rin, Gin and Bin that are inputted to the color discrimination circuit 47 according to the Modification 2-1 of the second embodiment of the present invention; and
Fig. 11 is a configuration diagram illustrating a configuration of a capsule endoscope system according to a third embodiment of the present invention.

Best Mode for Carrying Out the Invention

Embodiments of the present invention are described hereunder with reference to the drawings.

(First Embodiment)

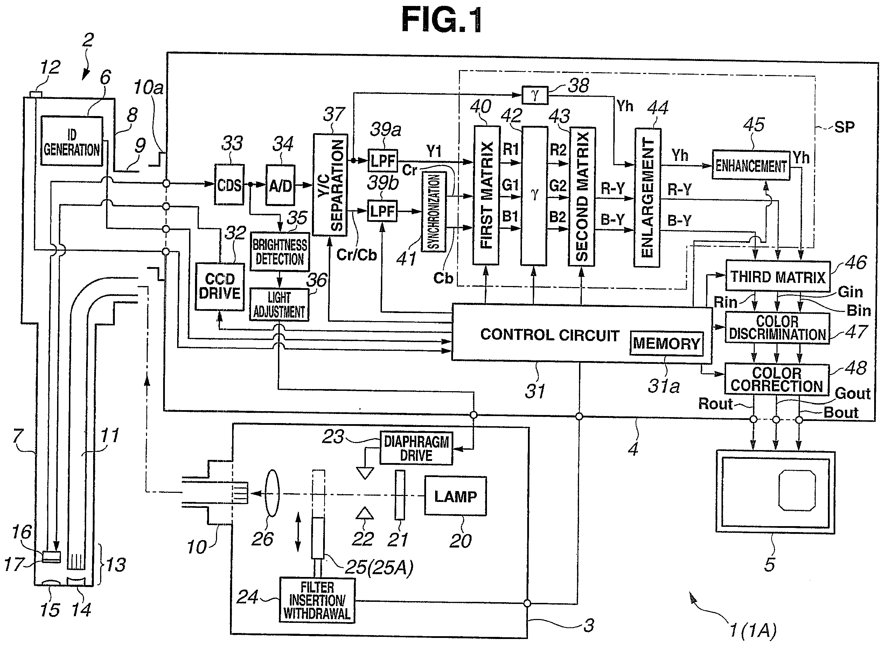

Fig. 1 is a view illustrating the configuration of an endoscope apparatus 1 according to a first embodiment. The endoscope apparatus 1 of the first embodiment shown in Fig. 1 includes an electronic endoscope (hereunder, referred to simply as "endoscope") 2 that is inserted into a body cavity to perform endoscopy, and a light source apparatus 3 that supplies illuminating light to the endoscope 2. The endoscope apparatus 1 that is a medical instrument also includes: a video processor (hereunder, referred to as "processor") 4 as an endoscope video signal processing apparatus that drives image pickup means incorporated in the endoscope 2 and performs signal processing on an output signal of the image pickup means; and a monitor 5 into which is inputted a video signal that is outputted from the processor 4, and which displays an image obtained by applying signal processing to an image picked up by the image pickup means as an endoscopic image.

The endoscope 2 includes an ID generation portion 6 that generates identification information (ID) that is unique to the endoscope 2, an elongated insertion portion 7, an operation portion 8 provided at a rear end of the insertion portion 7, and a universal cable 9 that is extended from the operation portion 8. A light guide connector 10 at one end portion of the universal cable 9 is detachably connected to the light source apparatus 3, and a signal connector 10a at another end is detachably connected to the processor 4 that is also a medical instrument.

A light guide 11 that transmits illuminating light is inserted through the inside of the insertion portion 7, and by connecting the light guide connector 10 at the end portion on the user's hand side of the light guide 11 to the light source apparatus 3, illuminating light from the light source apparatus 3 is supplied to the light guide 11.

In an observation mode that uses normal light that is white color light (hereunder, referred to as "white-color-light observation mode"), the light source apparatus 3 generates white color illuminating light that covers a visible wavelength region as the illuminating light, and supplies the white color illuminating light to the light guide 11. In a narrow band observation mode that is an observation mode that uses special light, the light source apparatus 3 generates illuminating light of a predetermined narrow band as the illuminating light and supplies the generated illuminating light to the light guide 11.

An instruction for switching of the white-color-light observation mode and the narrow band observation mode can be made, for example, by means of a mode switching switch 12 that is constituted by a scope switch or the like provided in the operation portion 8 of the endoscope 2. Note that apart from being constituted by a scope switch provided in the endoscope 2, the mode switching switch 12 may be constituted by a foot switch, a mode switching switch may be provided on a front panel of the processor 4, or the mode switching switch 12 may be constituted by an unshown keyboard or the like.

A switching signal from the mode switching switch 12 is inputted to a control circuit (described later) inside the processor 4. When the switching signal is inputted, the control circuit controls a filter insertion/withdrawal mechanism (described later) of the light source apparatus 3 to selectively switch between white color illuminating light and narrow band illuminating light.

An illumination lens 14 constituting illumination means that is mounted to an illuminating window is provided in the distal end portion 13 of the insertion portion 7. Illuminating light from the light source apparatus 3 is transmitted through the light guide 11 to the distal end face thereof, and passes through the illumination lens 14 provided in the distal end portion 12 of the insertion portion 7 and is emitted to outside to illuminate the surface of living tissue of a diseased part inside a body cavity or the like. As described above, the light source apparatus 4 and the light guide 11 and the like constitute illumination means that switchably irradiates normal light and special light.

Further, in the distal end portion 13, an observation window is provided adjacent to the illuminating window, and an objective lens 15 is mounted to the observation window. The objective lens 15 forms an optical image by means of reflected light from living tissue. A CCD 16 as a solid image pickup device constituting image pickup means is disposed at an image formation position of the objective lens 15, and light that passes through the objective lens 15 is subjected to photoelectric conversion by the CCD 16.

A color separation filter 17 that performs optical color separation is provided on an image pickup face of the CCD 16, and for example, a complementary color filter is mounted in respective pixel units on the color separation filter 17.

In the complementary color filter, four color chips of magenta (Mg), green (G), cyan (Cy) and yellow (Ye) are disposed in front of the respective pixels, with Mg and G being alternately disposed in the horizontal direction, and an array of Mg, Cy, Mg, Ye and an array of G, Ye, G, Cy being disposed in that order, respectively, in the vertical direction.

The light source apparatus 3 incorporates a lamp 20 that generates illuminating light. The lamp 20 generates illuminating light that includes a visible wavelength region. Infrared light of the illuminating light is cut off by an infrared cut-off filter 21 to obtain illuminating light of a wavelength close to a wavelength band of substantially white color light, and thereafter the illuminating light is made incident on a diaphragm 22. An opening amount of the diaphragm 22 is adjusted by a diaphragm drive circuit 23 to control the quantity of light passing through the diaphragm 22.

The illuminating light that has passed through the diaphragm 22 is condensed by a condensing lens 26 after passing through a narrow-band filter 25 that is inserted into or withdrawn from an illuminating light path by a filter insertion/withdrawal mechanism 24 constituted by a plunger or the like in the narrow band observation mode or without passing through the narrow-band filter 25 in the white-color-light observation mode, and is made incident on an end face on the user's hand side of the light guide 11, that is, on an incident end face.

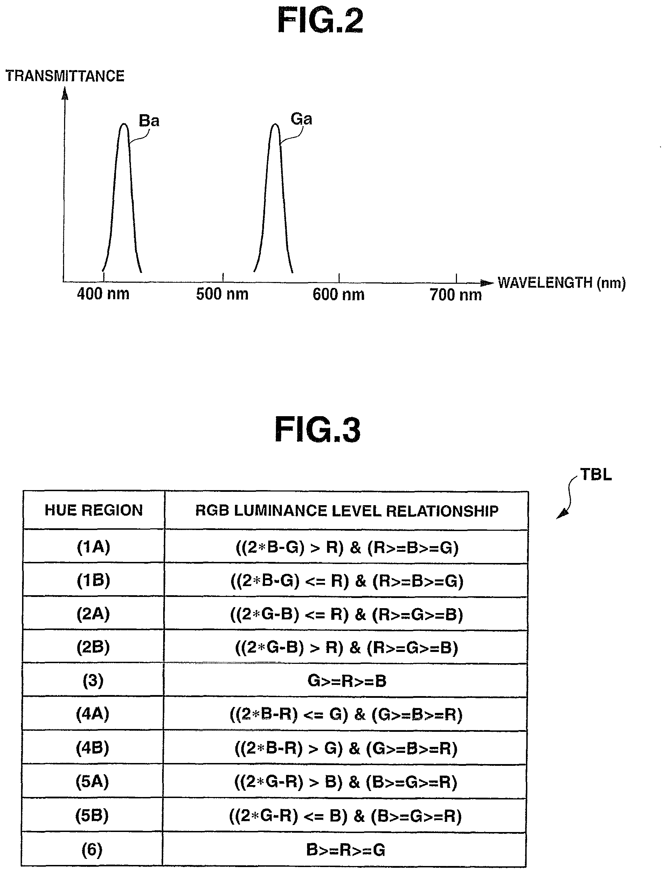

Fig. 2 is a view illustrating an example of spectral characteristics of the narrow-band filter 25. The narrow-band filter 25 exhibits a dual-mode filter characteristic, and for example, includes narrow band transmission filter characteristic portions Ga and Ba for the wavelength regions of green and blue, respectively.

More specifically, the narrow band transmission filter characteristic portions Ga and Ba have band-pass characteristics in which the respective center wavelengths are 540 nm and 415 nm, and the full widths at half maximum are between 20 and 40 nm.

Therefore, when the narrow-band filter 25 is disposed in the illuminating light path, narrow band illuminating light of two bands that is transmitted through the narrow band transmission filter characteristic portions Ga and Ba is incident on the light guide 11.

In contrast, when the narrow-band filter 24 is not disposed in the illuminating light path, white color light of a wide band is supplied to the light guide 11.

The processor 4 is a processor for an endoscope that processes an endoscopic image, and includes a control circuit 31 and various circuits. The main circuits among the various circuits operate under the control of the control circuit. In conjunction with switching control of illuminating light supplied to the light guide 13 from the light source apparatus 3, the control circuit 31 also performs control that switches the characteristics of a signal processing system inside the processor 4. Hence, the processor 4 is configured to be capable of performing signal processing that is suited to the respective observation modes, i.e. the white color light mode and narrow band mode, by switching the characteristics of the signal processing system by a switching operation of the mode switching switch 12.

The CCD 16 is connected to one end of a signal wire. By connecting the signal connector 10a that is connected to the other end of the signal wire to the processor 4, a CCD drive circuit 32 and a CDS circuit 33 inside the processor 4 are connected.

Note that an ID signal of the ID generation portion 6 that generates unique identification information (ID) of the endoscope 2 is inputted to the control circuit 31, and based on the received ID signal the control circuit 31 identifies the kind of the endoscope 2 connected to the processor 4 and the number of pixels and kind of the CCD 16 contained in the endoscope 2 and the like. The control circuit 31 controls the CCD drive circuit 32 so as to appropriately drive the CCD 16 of the endoscope 2 that is identified.

The CCD 16 outputs an image pickup signal that has undergone photoelectric conversion by application of a CCD drive signal from the CCD drive circuit 32, to the CDS circuit 33 that performs correlated double sampling. A signal component is extracted from the image pickup signal by the CDS circuit 33, converted to a baseband signal, then inputted to an A/D conversion circuit 34 to be converted to a digital signal, and also inputted to a brightness detection circuit 35 where the brightness (average luminance of the signal) is detected.

The brightness signal detected by the brightness detection circuit 35 is inputted to a light-adjusting circuit 36 to generate a light adjustment signal for adjusting light based on a difference value with a reference brightness (target value of light adjustment). The light adjustment signal from the light-adjusting circuit 36 is inputted to the diaphragm drive circuit 23, and the diaphragm drive circuit 23 adjusts an opening amount of the diaphragm 22 so that a brightness of a generated image becomes the reference brightness.

A digital signal that is outputted from the A/D conversion circuit 34 is inputted to a Y/C separation circuit 37. The Y/C separation circuit 37 generates a luminance signal Y and line-sequential color difference signals Cr and Cb (as a color signal C in a broad sense). In the case of the CCD 16 using the complementary color filter for the color separation filter 17, pixels of two columns neighboring each other in the vertical direction are added and sequentially read out, and a configuration is adopted so that at such time pixels are read out by shifting the columns of pixels between an odd-numbered field and an even-numbered fields. A signal that is read out from the CCD 16 is inputted to the Y/C separation circuit 37, and a luminance signal and a color difference signal are generated for each pixel, as is known.

The Y/C separation circuit 37 forms color separation means, and therefore the luminance signal Y as the output signal of the Y/C separation circuit 37 corresponds to a luminance signal, and the color difference signals Cr and Cb correspond to color difference signals.

The luminance signal Y is inputted to a $\gamma$ (gamma) circuit 38 and is also inputted to a first low-pass filter (hereunder, abbreviated as "LPF") 39a for limiting the pass band of the signal.

The LPF 39a is set to have a broad pass band in accordance with the luminance signal Y, and a luminance signal YI in the band set in accordance with the pass-band characteristic of the LPF 39a is inputted to a first matrix circuit 40 as color conversion means.

In addition, the color difference signals Cr and Cb are inputted to a synchronization circuit 41 that synchronizes the line-sequential color difference signals, through a second LPF 39b for limiting the pass bands of the signal.

In this case, the pass-band characteristic of the second LPF 39b is changed by the control circuit 31 according to the observation mode. More specifically, in the white-color-light observation mode, the second LPF 39b is set to a lower band than the first LPF 39a. That is, in the white-color-light observation mode, the second LPF 39b is set so as to perform signal processing in conformity with a typical video signal standard.

On the other hand, in the narrow band observation mode, the second LPF 39b is set to a broader band than the low band in the white-color-light observation mode. For example, the second LPF 39b is set, that is, changed, to a broad band that is approximately the same as that of the first LPF 39a.

Thus, the second LPF 39b forms processing characteristic changing means for changing processing characteristics to limit a pass band for the color difference signals Cr and Cb in conjunction with switching of the observation modes.

By widening the signal pass-band characteristic of the second LPF 39b, it is possible to improve the resolution of the running state of a capillary vessel or the running state of a blood vessel close to the vicinity of the surface layer obtained by a color signal of green (G), an image of which is picked up under illuminating light of green (G) that is close to a luminance signal from the narrow band transmission filter characteristic portion Ga, and obtain an image of good image quality that facilitates diagnosis.

The synchronization circuit 41 generates synchronized color difference signals Cr and Cb, and the color difference signals Cr and Cb are inputted to the first matrix circuit 40 as color conversion means.

The first matrix circuit 40 converts the luminance signal Yl and color difference signals Cr and Cb into three primary color signals R1, G1 and B1, and outputs the three primary color signals. The outputted three primary color signals R1, G1 and B1 are inputted to a $\gamma$ circuit 42 that performs gamma correction. Image signals obtained by irradiation of narrow band light having a center wavelength of 540 nm are assigned to the signals R1 and G1 among the three primary color signals, and an image signal obtained by irradiation of narrow band light having a center wavelength of 415 nm is assigned to the signal B1 among the three primary color signals.

The first matrix circuit 40 is controlled by the control circuit 31, and changes or switches the values of matrix coefficients which determine the conversion characteristic, according to a sensitivity characteristic of the CCD 16, the characteristic of the color separation filter 17 and the characteristic of the narrow-band filter 25. The first matrix circuit 40 converts the inputted signals to the three primary color signals R1, G1 and B1 that have no or almost no color mixture.

For example, the spectral sensitivity of the CCD 16 or the characteristic of the color separation filter 17 mounted in the endoscope 2 may differ depending on the endoscope 2 that is actually connected to the processor 4, and therefore the control circuit 31 changes the matrix coefficients for converting to the three primary color signals R1, G1 and B1 by means of the first matrix circuit 40 in accordance with the spectral sensitivity of the CCD 16 and the characteristic of the color separation filter 17 that are actually being used based on the information of the ID signal.

By adopting this configuration, even when the kind of image pickup means that is actually being used is different, it is possible to also appropriately correspond to the difference, and thus the occurrence of false colors can be prevented and the luminance signal Yl and color difference signals Cr and Cb can be converted to three primary color signals R1, G1 and B1 that have little color mixture.

Note that the control circuit 31 includes a memory 31a as a storage portion that stores data of various tables for reference that are referred to for determining matrix coefficients by the first matrix circuit 40 and a second matrix circuit and third matrix circuit that are described later.

The $\gamma$ circuit 42 is also controlled by the control circuit 31. More specifically, in the narrow band observation mode, the characteristic of $\gamma$ correction is modified to a $\gamma$ characteristic that is emphasized more than in the white-color-light observation mode. As a result, the contrast on the low signal level side is emphasized and provides a display characteristic that is easier to distinguish.

Three primary color signals R2, G2 and B2 that have undergone $\gamma$ correction by the $\gamma$ circuit 42 are inputted to a second matrix circuit 43 that constitutes color conversion means. The second matrix circuit 43 converts the primary color signals R2, G2 and B2 to color difference signals R-Y and B-Y using equation 1 below, and outputs the color difference signals R-Y and B-Y. Note that, for example, a matrix Mat1 of equation 1 is expressed as shown in equation 2.

$$\begin{bmatrix} R-Y \\ B-Y \end{bmatrix} = Mat1 \cdot \begin{bmatrix} R2 \\ G2 \\ B2 \end{bmatrix} \qquad \ldots \text{(equation 1)}$$

$$Mat1 = \begin{bmatrix} 0.496 & -0.453 & -0.043 \\ -0.113 & -0.383 & 0.496 \end{bmatrix} \qquad \ldots \text{(equation 2)}$$

The second matrix circuit 43 adopts, for example, matrix coefficients that are fixed to fixed values irrespective of switching between the observation modes.

The luminance signal Y that is inputted to the $\gamma$ (gamma) circuit 38 is subjected to gamma correction, and a resulting luminance signal Yh that has undergone gamma correction is inputted to an enlargement circuit 44.

The color difference signals R-Y and B-Y outputted by the second matrix circuit 43 are inputted to the enlargement circuit 44 together with the luminance signal Yh to undergo enlargement processing.

The luminance signal Yh that has undergone enlargement processing (and necessary interpolation processing) by the enlargement circuit 44 is subjected to edge enhancement by an enhancement circuit 45, and thereafter is inputted to a third matrix circuit 46. The color difference signals R-Y and B-Y that have undergone enlargement processing by the enlargement circuit 44 are inputted to the third matrix circuit 46 without passing through the enhancement circuit 45.

The luminance signal Yh and the color difference signals R-Y and B-Y are converted to three primary color signals Rin, Gin and Bin by the third matrix circuit 46 as color separation means. That is, the third matrix circuit 46 generates three primary color signals Rin, Gin and Bin from the luminance signal Yh and the color difference signals R-Y and B-Y.

In the white-color-light observation mode, the third matrix circuit 46 generates the three primary color signals Rin, Gin and Bin from the luminance signal Yh and the color difference signals R-Y and B-Y so as to generate a normal image that is obtained by irradiation with normal light, that is, white color light.

In the narrow band observation mode, to enable observation of a blood vessel image or a microstructure of a mucous membrane with good contrast, the third matrix circuit 46 assigns an image signal that is based on illumination of narrow band light having a center wavelength of 540 nm to the signal Rin, and assigns image signals based on illumination of narrow band light having a center wavelength of 415 nm to the signal Bin and signal Gin, and outputs the image signals.

Conversion from the luminance signal Yh and the color difference signals R-Y and B-Y to the three primary color signals Rin, Gin and Bin by the third matrix circuit 46 is performed by means of equation 4 below. Note that a matrix Mat 2 of equation 4 is a matrix for converting from the luminance signal Yh and the color difference signals R-Y and B-Y to the three primary color signals R, G and B, and more specifically is expressed as shown in equation 3.

$$
\text{Mat2} = \begin{bmatrix} 0.211 & 0.715 & 0.070 \\ 0.496 & -0.453 & -0.043 \\ -0.113 & -0.383 & 0.496 \end{bmatrix}^{-1} = \begin{bmatrix} 1.004 & 1.588 & -0.005 \\ 1.004 & -0.469 & -0.183 \\ 1.004 & 0.001 & 1.874 \end{bmatrix} \quad \dots \text{(equation 3)}
$$

A matrix Mat3 of equation 4 is a matrix for generating a reproduced color of living tissue from the three primary color signals R, G and B, and in the NBI mode, for example, is a matrix shown in equation 5, and in the white-color-light observation mode is expressed by a unit matrix of 3 rows by 3 columns. According to the matrix Mat3, an image signal based on illumination of narrow band light having a center wavelength of 540 nm is assigned to the signal Rin that is outputted from the third matrix circuit 46, and image signals based on illumination of narrow band light having a center wavelength of 415 nm are assigned to the signals Bin and Gin.

$$
\begin{bmatrix} \text{Rin} \\ \text{Gin} \\ \text{Bin} \end{bmatrix} = \text{Mat3} \cdot \text{Mat2} \cdot \begin{bmatrix} \text{Yh} \\ \text{R} - \text{Y} \\ \text{B} - \text{Y} \end{bmatrix} \quad \dots \text{(equation 4)}
$$

$$
\text{Mat3} = \begin{bmatrix} 0 & \text{m12} & 0 \\ 0 & 0 & \text{m23} \\ 0 & 0 & \text{m33} \end{bmatrix} \quad \dots \text{(equation 5)}
$$

If the output of the third matrix circuit 46 is outputted as it is to the monitor 5, an object other than living tissue such as residue or intestinal juice that is reproduced in a yellow color in the white-color-light observation mode will be displayed in a deep red color. Therefore, in this case, by processing performed by a color discrimination circuit 47 and a color conversion circuit 48, this kind of object that is other than living tissue is displayed in a color tone that is similar to a color displayed in the white-color-light observation mode.

As described above, the Y/C separation circuit 37 and the first to third matrix circuits 40, 43 and 46 and the like of the processor 4 constitute image signal generation means that generates image signals of normal light and image signals of special light from an output of the CCD 16 that picks up an image of returning light of light irradiated onto living tissue by illuminating light from the light source apparatus 4 that is illumination means.

The output of the third matrix circuit 46 is inputted to the color discrimination circuit 47. The color discrimination circuit 47 refers to a table TBL in accordance with luminance levels of the respective signals Rin, Gin and Bin that are inputted from the third matrix circuit 46, and determines which hue region each pixel is included in. The table TBL may be included in the color discrimination circuit 47, or a configuration may be adopted in which the table TBL is stored in the memory 31a of the control circuit 31, and the color discrimination circuit 47 can refer to the table TBL.

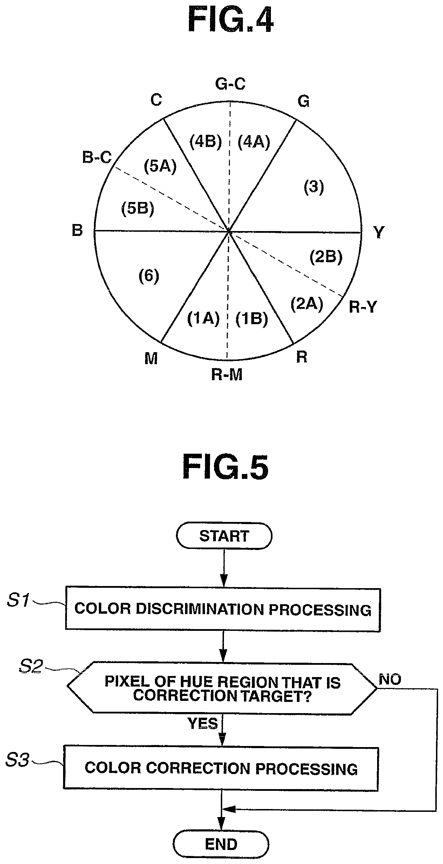

Fig. 3 is a view that illustrates that configuration of the table TBL that stores hue regions that are discriminated by the color discrimination circuit 47 and a discrimination reference thereof. Fig. 4 is a view for describing color spaces that are discriminated by the color discrimination circuit 47 and color correction processing thereof.

The table TBL shown in Fig. 3 is a table for defining ten hue regions based on a magnitude relationship among the R signal, G signal and B signal. The table TBL includes information regarding a correlation between a magnitude relationship of the respective luminance levels of R, G and B and the hue regions in a case that satisfies that relationship. Based on the table TBL shown in Fig. 3, it is determined whether the respective pixels belong to any one of the ten hue regions (1A), (1B), (2A), (2B), (3), (4A), (4B), (5A), (5B), and (6).

Color axes that are set radially from the center point of the color space shown in Fig. 4 represent the intensity of chroma (hereunder, also referred to as "color saturation" or simply as "saturation", and denoted by the reference symbol "sat"), and indicate that the color saturation increases outwardly from the center of a color circle. Further, a circumferential direction of the color space represents hue (hereunder, denoted by the reference symbol "hue").

The color space shown in Fig. 4 includes ten color axes, namely four color axes G-C, B-C, R-M, and R-Y in addition to six reference color axes C (cyan), B (blue), M (magenta), R (red), Y (yellow) and G (green). The color axis G-C is set between the reference color axes G and C, the color axis B-C is set between the reference color axes B and C, the color axis R-M is set between the reference color axes R and M, and the color axes R-Y is set between the reference color axes R and Y. In Fig. 4, hue regions into which a color space is divided by the ten color axes correspond to (1A), (1B), (2A), (2B), (3), (4A), (4B), (5A), (5B) and (6) that are discriminated in Fig. 3. For each pixel, the color discrimination circuit 47 discriminates to which hue region of Fig. 4 the relevant pixel belongs, using table TBL. Hence, the color discrimination circuit 47 discriminates a hue for each pixel in accordance with the luminance level of an image signal of special light.

As described above, an object that is reproduced in a yellow color under the white-color-light observation mode is reproduced in a red color tone with a high luminosity and chroma in the output of the third matrix circuit 47 in the narrow band observation mode.

Therefore, the color correction circuit 48 performs color correction with respect to pixels discriminated as belonging to the hue regions 2A and 1B. A pixel discriminated as belonging to the hue region 1B or a pixel determined as belonging to the hue region 2A is corrected in the direction of the color axis Y. That is, when the color discrimination circuit 47 determines that an observation object other than the living tissue has a red color tone, the color correction circuit 48 performs color correction to a yellow color tone.

Further, as described above, an object that is reproduced in blue under a white-color-light observation mode is reproduced in a green or blue-green color tone with a high luminosity and chroma in the output of the third matrix circuit 46 under a conventional narrow band observation mode.

Therefore, the color correction circuit 48 performs color correction also with respect to pixels discriminated as belonging to color spaces from hue region 4A to 4B and 5A. A pixel discriminated as belonging to a color space from hue region 5A to 4B or a pixel determined as belonging to hue region 4A is corrected in the direction of color axis B. That is, when the color discrimination circuit 47 discriminates that an observation object other than living tissue is a green color tone or a blue-green color tone, the color correction circuit 48 performs color correction to a blue color tone.

As described above, the color discrimination circuit 47 constitutes color discrimination means that discriminates a color for each pixel with respect to an image signal of special light. More specifically, the color discrimination circuit 47 constitutes color discrimination means that identifies a color of each pixel with respect to an image signal of special light, and discriminates whether or not the observation object is other than living tissue. In addition, the color correction circuit 48 constitutes color correction means that, in an observation mode that uses special light, based on the discrimination result of the color discrimination circuit 47, performs color correction with respect to a color of an observation object that is other than living tissue so that the color is similar to a color at a time of an observation mode that uses normal light.

Fig. 5 is a flowchart illustrating color discrimination and color correction processing that is performed for each pixel in the color discrimination circuit 47 and the color correction circuit 48. In this case, the color correction processing is described taking a method described, for example, in International Publication No. WO 2010/044432 as an example.

The color discrimination circuit 47 refers to the table TBL based on the signals Rin, Gin and Bin for the respective pixels that are inputted from the third matrix circuit 46, and determines whether or not the relevant pixel belongs to a hue region thereof (S1).

Based on the determination result of the color discrimination circuit 47, the color correction circuit 48 determines whether or not the relevant pixel is a pixel of a hue region that is a correction target that should be subjected to color correction (S2). If the relevant pixel is a pixel of a hue region that is a correction target (S2: Yes), the color correction circuit 48 performs predetermined color correction (S3). If the relevant pixel is not a pixel of a hue region that is a correction target (S2: No), the color correction circuit 48 does not perform predetermined color correction.

In this case, among the ten hue regions, the hue regions for which color correction is performed are the hue regions 2A, 1B, 4A, 4B and 5A.

Based on equation 6 below, the color correction circuit 48 performs color correction of pixels, and outputs corrected signals Rout, Gout, Bout of the relevant pixel.

$$
\left.\begin{array}{l}
Rout = Rin + p_{sat} + (p_{hue} \times R_{-a1}) + c_{sat} + (c_{hue} \times R_{-a2}) \\
Gout = Gin + p_{sat} + (p_{hue} \times G_{-a1}) + c_{sat} + (c_{hue} \times G_{-a2}) \\
Bout = Bin + p_{sat} + (p_{hue} \times B_{-a1}) + c_{sat} + (c_{hue} \times B_{-a2})
\end{array}\right\} \quad \dots \text{(equation 6)}
$$

Here, correction coefficients $P_{sat}$, $P_{hue}$, $C_{sat}$ and $C_{hue}$ are calculated based on equation 7 below, and correction coefficients $R_{-a1}$, $G_{-a1}$, $B_{-a1}$, $R_{-a2}$, $G_{-a2}$ and $R_{-a2}$ are fixed values.

$$
\left.\begin{array}{l}
p_{sat} = K_{sat1} \times d_p \\
p_{hue} = K_{hue1} \times d_p \\
c_{sat} = K_{sat2} \times d_c \\
c_{hue} = K_{hue2} \times d_c
\end{array}\right\} \quad \dots \text{(equation 7)}
$$

In equation 7, coefficients $d_p$ and $d_c$ are variables that are calculated based on the pixel value of the relevant pixel, and coefficients $K_{sat1}$, $K_{hue1}$, $K_{sat2}$ and $K_{hue2}$ are correction coefficients applied to the hue region in which a color signal of the relevant pixel is located that are fixed values.

The correction coefficients $K_{sat1}$, $K_{hue1}$, $K_{sat2}$ and $K_{hue2}$ are coefficients that determine an amount of movement or a conversion amount on a color space. That is, the correction coefficients $K_{sat1}$, $K_{hue1}$, $K_{sat2}$ and $K_{hue2}$ determine an amount by which pixels of the hue regions 2A, 1B, 4A, 4B and 5A for which color correction is performed are moved or rotated on the respective color spaces.

There are three values for the four correction coefficients $K_{sat1}$, $K_{hue1}$, $K_{sat2}$ and $K_{hue2}$, respectively, that are used for the hue regions 1B and 2A, hue regions 4B and 5A, and hue regions 4A and 3, and the values are selected in accordance with the hue region in which the color signal of the relevant pixel is located. The processing shown in Fig. 5 is performed for each pixel.

As described above, since the hue of an object belonging to the hue region 1B or 2A that appears in a yellow color under white-color-light observation such as residue is rotated on the color space in the yellow direction (that is, color correction is performed) by the discrimination circuit 47 and the color correction circuit 48, the object is displayed in yellow or orange on the monitor 5. Further, since the hue of a staining agent that appears blue under white-color-light observation such as indigo carmine or methylene blue and belongs to the hue region 4B, 5A or 5B is rotated on the color space in the blue direction (that is, color correction is performed), the staining agent is displayed in blue on the monitor 5.

Hence, under the narrow band observation mode, a surgeon can see an object other than living tissue in a color tone that is similar to a color thereof in an image displayed in a white color light mode. In particular, since residue or the like is not seen in a deep red color and a blue pigment is not seen in a hue region from green to blue-green, a situation in which a surgeon momentarily misidentifies such an object that is other than living tissue or in which a surgeon feels a sense of incongruity when viewing an image obtained in the narrow band observation mode can be eliminated. Consequently, for example, the surgeon can smoothly perform an examination or the like using an endoscope.

Note that, although in the above described example the correction coefficients $K_{sat1}$, $K_{hue1}$, $K_{sat2}$ and $K_{hue2}$ are fixed values, the correction coefficients may be variables.

For example, a configuration may also be adopted that changes the correction coefficients $K_{sat1}$, $K_{hue1}$, $K_{sat2}$ and $K_{hue2}$ based on the signals R1, G1 and B1 that are outputted from the first matrix circuit 42. More specifically, $\alpha 1$ is calculated by equation 8 below based on the signals R1, G1 and B1 outputted from the first matrix circuit 42.

$$
\alpha 1 = \frac{|G1 - B1|}{G1} \quad \dots \text{(equation 8)}
$$

The value of $\alpha 1$ is proportional to a difference between the signals G1 and B1 (or a difference between the signals R1 and B1). Hence, the correction coefficients $K_{sat1}$, $K_{hue1}$, $K_{sat2}$ and $K_{hue2}$ are changed in proportion to $\alpha 1$. As

a result, since the level of chroma is high when a difference between the signals G1 and B1 is large, the correction coefficients $K_{sat1}$, $K_{hue1}$, $K_{sat2}$ and $K_{hue2}$ are changed so as to correct the amount of movement on the color space by a larger degree. That is, with respect to a color of an observation object other than living tissue, the color correction circuit 48 dynamically changes correction parameters in the color correction processing in accordance with a luminance level of an image signal of special light from the CCD 16.

Accordingly, with respect to the color of a pixel, if the chroma of green or blue-green is high, the color of the relevant pixel is corrected so as to become bluer, while if the chroma of red is high, the color of the relevant pixel is corrected so as to become more yellow.

(Modification 1)

In the above described example, the light source apparatus 3 is configured so as to emit white color light in the white-color-light observation mode and emit predetermined narrow band light in the narrow band observation mode, and to receive the returning light thereof with an image pickup device. However, as a modification thereof, a light source apparatus as illumination means may be configured to emit only normal light that is white color light, and in a narrow band observation mode, to generate an image signal that corresponds to returning light of a narrow band light that is special light by so-called "spectral estimation processing" that is known processing. With regard to spectral estimation processing, for example, Japanese Patent Application Laid-Open Publication No. 2003-93336 discloses an electronic endoscope apparatus configured to perform signal processing on an image signal acquired using illuminating light in a visible light region and generate a discrete spectral image, and obtain image information of living tissue.

In the case of Modification 1, the light source apparatus 3 shown in Fig. 1 need not include the narrow-band filter 25 and the filter insertion/withdrawal mechanism 24, and in addition, a circuit portion SP in the area indicated by a dotted line is a processing portion that performs spectral estimation processing. The circuit portion SP is constituted by a CPU or a DSP or the like. In the circuit portion SP, after performing conversion processing from a complementary color system to a primary color system, spectral estimation processing is performed. The configuration and processing contents other than the configuration and processing contents of the circuit portion SP are the same as in the above described example.

Hence, a configuration may also be adopted that generates a signal of returning light of discrete narrow band light using the above described spectral estimation processing.

(Modification 2)

Although in the above example the color discrimination circuit performs color discrimination based on the RGB colorimetric system, a configuration may also be adopted so as to perform color discrimination based on another colorimetric system, such as the CIE L*a*b* (L star, a star, b star) colorimetric system or the LUT colorimetric system.

For example, color discrimination can also be performed using a hue angle $\theta$ in the CIE L*a*b* colorimetric system that is one UCS (uniform color space). In this case, in a table similar to the table TBL shown in Fig. 4, a plurality of hue regions are defined based on the hue angle, it is determined whether or not each pixel belongs to a predetermined region within the defined plurality of hue regions, and correction of colors is performed as described above. For example, color correction processing is performed only with respect to pixels for which a hue angle in the L*a*b* colorimetric system belongs within a range of -45 degrees to 45 degrees or the like.

In addition, a configuration may also be adopted in which color discrimination is performed based on the hue angle $\theta$ and the chroma (a distance OC from the origin). In such a case, in a table that is similar to the table TBL shown in Fig. 4, a plurality of hue regions are defined based on the range of a hue angle and a chroma value, it is determined whether or not each pixel belongs to a predetermined region within the defined plurality of hue regions, and correction of colors is performed as described above.

Further, although in the above example the color correction circuit 48 performs 10-axis color correction as shown in the above equation 6 and equation 7, when using the L*a*b* colorimetric system, a configuration may be adopted so as to perform color correction by matrix calculation using equation 9 below with respect to signals Rin, Gin and Bin that are outputted from the third matrix circuit 46 that are taken as signals of an object other than living tissue discriminated as described above. A matrix Mat4 of equation 9, for example, is expressed by a matrix of 3 rows by 3 columns as shown in equation 10.

$$\begin{bmatrix} \text{Rout} \\ \text{Gout} \\ \text{Bout} \end{bmatrix} = \text{Mat4} \cdot \begin{bmatrix} \text{Rin} \\ \text{Gin} \\ \text{Bin} \end{bmatrix} \qquad \cdots \text{(equation 9)}$$

$$Mat4 = \begin{bmatrix} m11 & 0 & 0 \\ m21 & m22 & 0 \\ 0 & 0 & m33 \end{bmatrix} \qquad \ldots \text{(equation 10)}$$

Here, elements m21 and m22 of the matrix Mat4 are previously set so that a signal Rout and a signal Gout become approximately equal, that is, so that Rout ≈ Gout. As described above, the color correction circuit 48 performs color correction processing by matrix calculation using a matrix of correction coefficients with respect to the color of an observation object other than living tissue.

Note that when carrying out hue discrimination based on the hue angle θ and chroma, the elements m21 and m22 in equation 10 are changed to values that are previously prepared in accordance with the chroma.

(Modification 3)

In the above described example, in the narrow band observation mode, for example, to enable observation of a blood vessel image or a microstructure of a mucous membrane with good contrast, color conversion is performed at the third matrix circuit 46, and thereafter color correction is performed for pixels of a predetermined hue region by the color discrimination circuit 47 and the color conversion circuit 48. However, the order of the processing for color conversion by the third matrix circuit 46 and for color correction by the color discrimination circuit 47 and the color conversion circuit 48 may also be interchanged.

That is, a configuration may be adopted in which processing for color conversion by the third matrix circuit 46 is performed after performing processing for color correction by means of the color discrimination circuit 47 and the color conversion circuit 48.

As described in the foregoing, according to the above described embodiment and respective modifications, a medical instrument can be provided that, under a special-light observation image mode, displays an object other than living tissue in a color tone that is similar to a color thereof in an image that is displayed in a white-color-light observation mode.

(Second Embodiment)

Although light of two narrow bands are used according to the above described first embodiment, according to the present embodiment three narrow bands of light are used in order to display an object other than living tissue in a more distinguishable manner. In particular, although according to the above described first embodiment there are cases in which not only residue but also blood vessels, particularly capillary vessels, through which blood which includes hemoglobin flows are displayed in the same hue as residue, according to the present embodiment, capillary vessels and residue are reproduced in different hues and color correction processing is performed by changing matrix calculation contents based on the reproduced hues. Hence, capillary vessels and residue can be displayed in different hues.

Although the configuration of an endoscope apparatus 1A of the present embodiment is similar to the configuration of the endoscope apparatus 1 shown in Fig. 1, the processing and operations of some components are different. The endoscope apparatus of the present embodiment is described below using Fig. 1. Hereunder, a description of components that are the same as components of the endoscope apparatus 1 of the first embodiment is omitted, and processing and operations that are different from the first embodiment are mainly described.

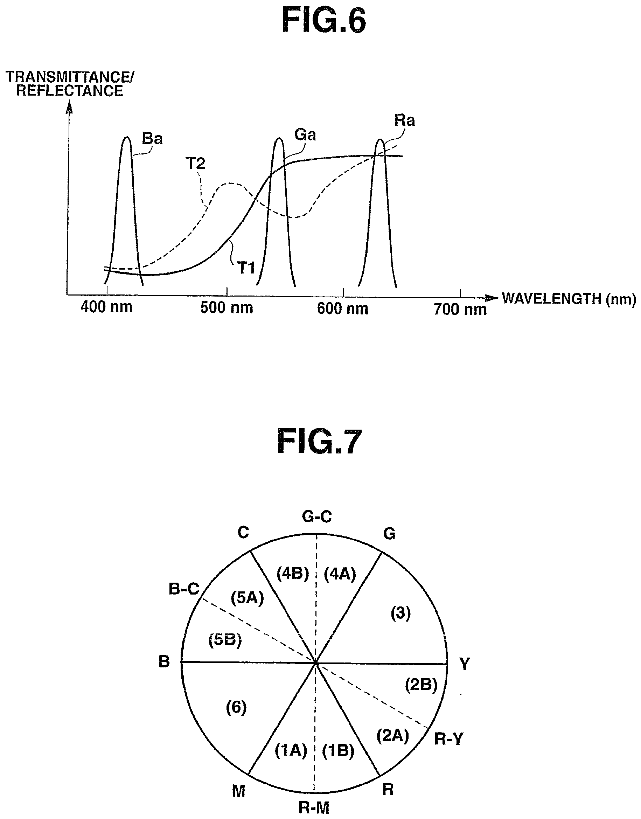

In the endoscope apparatus 1 of Fig. 1, the narrow-band filter 25A is a filter that transmits three narrow bands of light. Fig. 6 is a view that illustrates an example of spectral characteristics of the narrow-band filter 25A and reflectance of objects according to the second embodiment. The narrow-band filter 25A exhibits a tri-mode filter characteristic and, for example, includes narrow band transmission filter characteristic portions Ra, Ga and Ba for the wavelength regions of red, green and blue, respectively.

More specifically, the narrow band transmission filter characteristic portions Ra, Ga and Ba have band-pass characteristics in which the respective center wavelengths are 630 nm, 540 nm and 415 nm, and the full widths at half maximum are between 20 to 40 nm.

Therefore, when the narrow-band filter 25A is disposed in the illumination light path, narrow band illuminating light of three bands that is transmitted through the narrow band transmission filter characteristic portions Ra, Ga and Ba is incident on the light guide 11.

Reflected light from living tissue is received by the CCD 16, and image signals obtained by photoelectric conversion at the CCD 16 are inputted to the CDS circuit 33.

The first matrix 40 outputs an image signal that is based also on irradiation of narrow band light having a center wavelength of 630 nm as a signal R1, outputs an image signal that is based also on irradiation of narrow band light having a center wavelength of 540 nm as a signal G1, and outputs an image signal that is based also on irradiation of narrow band light having a center wavelength of 415 nm as a signal B1.

Further, the third matrix circuit 46 generates and outputs three primary color signals Rin, Gin and Bin based on the luminance signal Yh and color difference signals R-Y and B-Y. The signal Rin corresponds to the image signal that is based also on irradiation of narrow band light having a center wavelength of 630 nm, the signal Gin corresponds to the image signal that is based also on irradiation of narrow band light having a center wavelength of 540 nm, and the signal Bin corresponds to the image signal that is based also on irradiation of narrow band light having a center wavelength of 415 nm. Hence, according to the present embodiment, the third matrix 48 performs conversion processing that simply converts from the luminance signal Yh and color difference signals R-Y and B-Y to RGB signals.

The color discrimination circuit 47 is the same as in the above first embodiment, and refers to the table TBL shown in Fig. 3 to discriminate a hue region of each pixel.

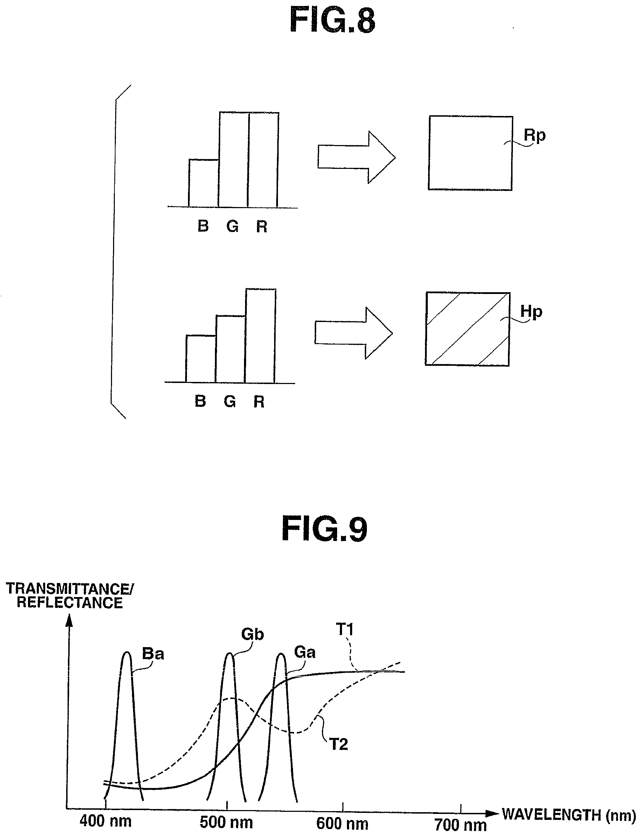

The reflectance of residue and hemoglobin will now be described using Fig. 6 by taking a difference between the reflectance of residue and hemoglobin as a concept. In Fig. 6, the reflectance of residue is shown by a curve T1 and the reflectance of hemoglobin Hb is shown by a curve T2. As shown in Fig. 6, although the reflectance of hemoglobin Hb is lower than the reflectance of residue in a wavelength band of narrow band light having a center wavelength of 540 nm, the reflectances of hemoglobin Hb and residue are substantially the same in a wavelength band of narrow band light having a center wavelength of 630 nm. The present embodiment is configured so as to improve the identification accuracy with respect to hemoglobin Hb and residue by utilizing such reflectance characteristics. That is, when an image of residue is picked up, since the relationship between the intensities of the signals Rin, Gin and Bin that are inputted to the color discrimination circuit 47 is as shown in the upper section of Fig. 8, the residue is reproduced with a yellowish hue. In contrast, when an image of a capillary vessel including hemoglobin Hb is picked up, the relationship between the intensities of the signals Rin, Gin and Bin inputted to the color discrimination circuit 47 is as shown in the lower section of Fig. 8, and the capillary vessel is reproduced with a red to orangish hue. Hence the reproduction hues of the residue and the capillary vessels differ, and it is relatively easy to discriminate between residue and capillary vessels based on the hues.

The color correction circuit 48 performs color correction processing that differs for each hue region that is discriminated by the color discrimination circuit 47. Fig. 7 is a view for describing color spaces discriminated by the color discrimination circuit 47 and color correction processing thereof according to the present embodiment.

According to the present embodiment, different correction processing is performed with respect to pixels belonging to hue regions 2B and 3, pixels belonging to hue regions 5B, 5A and 4B, and pixels belonging to hue regions other than the hue regions 2B, 3, 5A and 4B, respectively.

Color correction is performed by matrix calculation using equation 11 below with respect to pixels belonging to the hue regions 2B and 3. A matrix Mat5 of equation 11, for example, is expressed by a matrix of 3 rows by 3 columns as shown in equation 12.

$$\begin{bmatrix} \text{Rout} \\ \text{Gout} \\ \text{Bout} \end{bmatrix} = \text{Mat5} \cdot \begin{bmatrix} \text{Rin} \\ \text{Gin} \\ \text{Bin} \end{bmatrix} \qquad \text{... (equation 11)}$$

$$\text{Mat5} = \begin{bmatrix} 0 & \text{m12} & 0 \\ 0 & \text{m22} & \text{m23} \\ 0 & 0 & \text{m33} \end{bmatrix} \qquad \text{... (equation 12)}$$

Here, elements m22 and m23 are set so that signals Rout and Bout become approximately equal, and the elements have a relationship such that m22 = $\alpha \cdot$ m12, and m23 = (1-$\alpha$) $\cdot$ m23', where $\alpha$ is a value such that $0 \leq \alpha \leq 1$.

As shown in equation 11, since the matrix Mat5 includes the element m22 that is not 0 (zero), signals Rout and Gout are output signals that are in accordance with a difference between the reflectance of residue and the reflectance of hemoglobin Hb.

Since the reflectance of residue in a wavelength band of narrow band light having a center wavelength of 540 nm is greater than the reflectance of hemoglobin Hb, residue that is displayed on the monitor 5 is displayed in a yellower color by means of the signals Rout and Gout.

Fig. 8 is a view for describing differences in intensity with respect to residue and hemoglobin Hb, respectively, in signals Rin, Gin and Bin that are inputted to the color discrimination circuit 47 according to the present embodiment. In Fig. 8, the upper section is a view that includes a graph for describing signal intensities for B, G and R of residue, and the lower section is a view that includes a graph for describing signal intensities for B, G and R of hemoglobin Hb.

Further, color correction is performed by matrix calculation using equation 12 below with respect to pixels belonging to hue regions 5B, 5A and 4B. A matrix Mat6 of equation 13, for example, is expressed by a matrix of 3 rows by 3 columns as shown in equation 14.

$$
\begin{bmatrix} Rout \\ Gout \\ Bout \end{bmatrix} = Mat6 \cdot \begin{bmatrix} Rin \\ Gin \\ Bin \end{bmatrix} \qquad \ldots \text{(equation 13)}
$$

$$
Mat6 = \begin{bmatrix} 0 & m12 & 0 \\ 0 & 0 & m23' \\ 0 & m32 & m33' \end{bmatrix} \qquad \ldots \text{(equation 14)}
$$

Here, in the case of three narrow bands of light, since a blue pigment is reproduced as pixels of hue regions 5B, 5A and 4B, by using matrix Mat6 of equation 14 with respect to pixels of these two hue regions, a blue pigment undergoes color correction from blue-green to blue and is displayed.

Further, color correction is performed by matrix calculation using equation 15 below with respect to pixels belonging to hue regions other than the hue regions 2B, 3, 5B, 5A and 4B. A matrix Mat7 of equation 15, for example, is expressed by a matrix of 3 rows by 3 columns as shown in equation 16.

$$
\begin{bmatrix} Rout \\ Gout \\ Bout \end{bmatrix} = Mat7 \cdot \begin{bmatrix} Rin \\ Gin \\ Bin \end{bmatrix} \qquad \ldots \text{(equation 15)}
$$

$$
Mat7 = \begin{bmatrix} 0 & m12 & 0 \\ 0 & 0 & m23' \\ 0 & 0 & m33' \end{bmatrix} \qquad \ldots \text{(equation 16)}
$$

Thus, according to the present embodiment, since three narrow bands of light are used to facilitate discrimination of objects other than living tissue by color tone, capillary vessels and residue can be displayed in different hues.

(Modification 2-1)

Although in the above described embodiment, narrow band light having a center wavelength of 630 nm is used as a third narrow band of light, according to the present modification the identification accuracy with respect to capillary vessels and residue can be improved using narrow band light having a center wavelength of 500 nm.

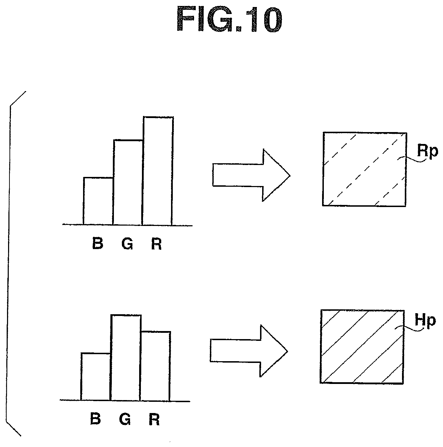

Fig. 9 is a view illustrating an example of spectral characteristics of a narrow-band filter 25A and reflectance of objects according to Modification 2-1 of the second embodiment. As shown in Fig. 9, in a wavelength band of narrow band light having a center wavelength of 500 nm the reflectance of hemoglobin Hb is higher than the reflectance of residue, and in a wavelength band of narrow band light having a center wavelength of 540 nm the reflectance of hemoglobin Hb is lower than the reflectance of residue. The present embodiment utilizes these reflectance characteristics to raise the accuracy of discrimination based on color tone with respect to capillary vessels and residue. That is, when an image of residue is picked up, since the relationship between the intensities of the signals Rin, Gin and Bin that are inputted to the color discrimination circuit 47 is as shown in the upper section of Fig. 10, the residue is reproduced with a red to orangish hue. In contrast, when an image of a capillary vessel including hemoglobin Hb is picked up, the relationship is as shown in the lower section of Fig. 10 and the capillary vessel is reproduced with a green to yellowish hue. Hence, the reproduction hues of residue and capillary vessels differ, and discrimination by hue is easy. Fig. 10 is a view for conceptually describing differences in intensity with respect to residue and hemoglobin Hb, respectively, in signals Rin, Gin and Bin that are inputted to the color discrimination circuit 47 according to Modification 2-1 of the present embodiment.

The color correction circuit 48 performs color correction processing that differs for each hue region that is discriminated by the color discrimination circuit 47.

Although color correction is performed with respect to pixels belonging to hue regions 2A and 2B by matrix calculation using equation 11 that is described above, for example, a matrix of 3 rows by 3 columns shown in equation 17 is used for the matrix Mat5 of equation 11.

$$\text{Mat5} = \begin{bmatrix} \text{m11} & 0 & 0 \\ \text{m21} & 0 & \text{m23''} \\ 0 & 0 & \text{m33} \end{bmatrix} \qquad \dots \text{(equation 17)}$$

Fig. 10 is a view for describing differences in intensity with respect to residue and hemoglobin Hb in signals Rin, Gin and Bin that are inputted to the color discrimination circuit 47. In Fig. 10, the upper section is a view that includes a graph for describing signal intensities for B, G and R of residue, and the lower section is a view that includes a graph for describing signal intensities for B, G and R of hemoglobin Hb. That is, a blood vessel including hemoglobin Hb is reproduced with a red to orangish hue, while residue is reproduced with a green to yellowish hue, and since the hues can be changed significantly, identification by hue can be enhanced.

In addition, color correction is performed by matrix calculation using the above described equation 13 with respect to pixels belonging to hue regions 5A and 4B, and for example, a matrix of 3 rows by 3 columns as shown in equation 18 below is used as the matrix Mat6 in equation 13.

$$\text{Mat6} = \begin{bmatrix} \text{m11} & 0 & 0 \\ 0 & 0 & \text{m23'''} \\ \text{m31} & 0 & \text{m33''} \end{bmatrix} \qquad \dots \text{(equation 18)}$$

Further, color correction is performed by matrix calculation using the above described equation 15 with respect to pixels belonging to hue regions other than the hue regions 2B, 3, 5A and 4B, and for example, a matrix of 3 rows by 3 columns as shown in equation 19 below is used as the matrix Mat7 in equation 15.

$$\text{Mat7} = \begin{bmatrix} \text{m11} & 0 & 0 \\ 0 & 0 & \text{m23'''} \\ 0 & 0 & \text{m33} \end{bmatrix} \qquad \dots \text{(equation 19)}$$

Thus, according to the present embodiment and the modification thereof, since three narrow bands of light are used so as to improve the accuracy of discriminating objects other than living tissue by color tone, capillary vessels and residue can be displayed in hues that are more different from each other.

Note that Modifications 1, 2 and 3 that are described in the first embodiment can also be applied to the second embodiment and Modification 2-1.

(Third Embodiment)

Although the medical instruments according to the two embodiments described above are endoscope apparatuses including an endoscope that has an insertion portion, a medical instrument of the present embodiment is an endoscope system that utilizes a swallow capsule endoscope. According to the present embodiment, with respect to images acquired by a capsule endoscope also, in a narrow band observation mode as described above it is possible to display an object other than living tissue in a color tone that is similar to a color thereof in an image displayed in a white-color-light observation mode.

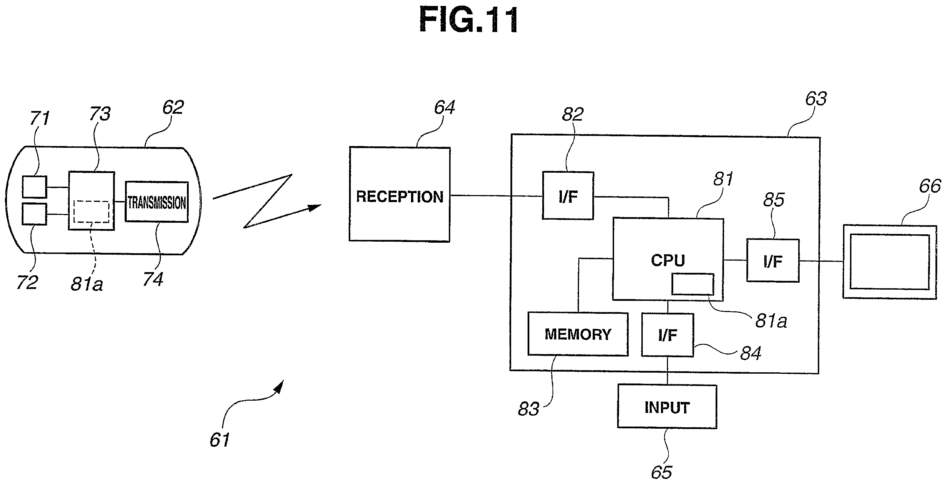

Fig. 11 is a configuration diagram that shows a configuration of a capsule endoscope system according to the present embodiment. A capsule endoscope system 61 includes a capsule endoscope 62, a work station 63 as a terminal apparatus, a reception portion 64 that receives an image signal from the capsule endoscope 62, an input portion 65 that is connected to the work station 63 and includes a keyboard and a mouse or the like, and a monitor 66 that displays an image that has been processed by the work station 63.

The capsule endoscope 62 includes an illumination portion 71, an image pickup portion 72, a control portion 73, and a transmission portion 74. The illumination portion 71 emits illuminating light that is white color light through an unshown illumination lens under the control of the control portion 73 to illuminate an observation object. The image pickup portion 72 includes an image pickup device such as a CCD and an objective lens, and under control of the control portion 73, picks up an image of returning light of the illuminating light and outputs an image pickup signal to the control portion 73.

The control portion 73 outputs the image signal to the transmission portion 74, and the transmission portion 74 that has an antenna transmits the image signal by radio transmission. The image signal from the capsule endoscope 62 is received by the reception portion 64 that has an antenna.

The work station 63 that is a processor for an endoscope that processes an endoscopic image includes a CPU 81 that is a central processing apparatus, an interface portion (I/F) 82 that interfaces with the reception portion 64, a memory 83, an interface portion (I/F) 84 that interfaces with the input portion 65, and an interface portion (I/F) 85 that interfaces with the monitor 66.

The capsule endoscope 62 transmits a digital image signal that is the same as the output of the A/D conversion circuit 34 shown in Fig. 1 based on an image pickup signal that is outputted by the image pickup device such as a CCD, by radio transmission from the transmission portion 74. The reception portion 64 supplies the received image signal to the work station 63, and the CPU 81 of the work station 63 receives the image signal through the interface portion 82. The work station 63 stores the received image signal in the memory 83.

The CPU 81 includes an image processing portion 81a. For each pixel from the received image pickup signal, the image processing portion 81a executes the spectral estimation portion processing described in Modification 1 of the first embodiment, color discrimination processing of the color discrimination circuit 47, and color correction processing of the color correction circuit 48. That is, the image processing portion 81a includes a spectral estimation processing portion, the color discrimination circuit 47, and the color correction circuit 48.

Under the special-light observation mode, since the CPU 81 outputs signals Rout, Gout and Bout that has been corrected by the color correction circuit 48, as described above, an object other than living tissue is displayed on the monitor 66 in a color tone that is similar to a color thereof in an image displayed in the white-color-light observation mode.

Accordingly, in the capsule endoscope system 61 also, an object other than living tissue can be displayed in a color tone that is similar to a color thereof in an image displayed in the white-color-light observation mode.

Note that, although in the above described example the image processing portion 81a is included in the work station 63, a configuration may also be adopted in which the image processing portion 81a is provided in the control portion 73 of the capsule endoscope 62 as shown by a dotted line in Fig. 11.

In this case, the capsule endoscope 62 transmits an image signal that has been corrected by the aforementioned color correction circuit 48 based on the image pickup signal that is outputted from the image pickup device such as a CCD, by radio transmission from the transmission portion 74. The reception portion 64 supplies the received image pickup signal to the work station 63, and the CPU 81 of the work station 63 receives the image signal through the interface portion 82. The work station 63 stores the received image signal in the memory 83.

Since the work station 63 receives the output signals Rout, Gout and Bout of the color correction circuit 48 of Fig. 1, under the special-light observation mode an object other than living tissue is displayed on the monitor 66 in a color tone that is similar to a color thereof in an image displayed in the white-color-light observation mode.

Moreover, although in the above described example the illumination portion 71 of the capsule endoscope 62 emits white color light, and a signal that corresponds to an image picked up as the result of irradiation of narrow band light is generated by spectral estimation at the control portion 73, a configuration may also be adopted in which the illumination portion 71 emits two or three narrow bands of light.

As described above, according to a medical instrument according to the foregoing three embodiments and the respective modifications thereof, in a special-light observation mode an object other than living tissue can be displayed in a color tone that is similar to a color thereof in an image displayed in a white-color-light observation mode.

Note that, although in the above described examples observation by means of returning light of narrow band light has been described as special-light observation, a special light may also be fluorescence with respect to excitation light or the like.

The present invention is not limited to the above described embodiments, and various changes and alterations can be made within a range that does not depart from the scope of the present invention.

The present application is filed claiming the priority of Japanese Patent Application No. 2011-207719 filed in Japan on September 22, 2011.

**Claims**

1. A medical instrument (1), comprising:

image signal generation means (37, 40, 43, 46) configured to generate an image signal of normal light and an image signal of narrow band light for observing a blood vessel image or a microstructure of a living body mucous membrane based on returning light of light that is irradiated onto living tissue by illumination means;

color discrimination means (47) configured to identify a color tone for respective pixels in accordance with a luminance level of an image signal of the narrow band light, and discriminate whether or not the respective pixels correspond to an observation object other than the living tissue; and

color correction means (48) configured to, in an observation mode that uses the narrow band light, based on the color discrimination means (47), if an observation object other than the living tissue is discriminated as being a red color tone, perform color correction to a yellow color tone, and if an observation object other than the living tissue is discriminated as being a green color tone or a blue-green color tone, perform color correction to a blue color tone.

2. The medical instrument (1) according to claim 1, wherein a luminance level of an image signal of the narrow band light is a luminance level of an RGB signal.

3. The medical instrument (1) according to claim 1, wherein:

the illumination means is configured to switchably irradiate the normal light and the narrow band light; and
the image signal generation means (37, 40, 43, 46) is configured to generate an image signal of the narrow band light that is irradiated onto the living tissue by the illumination means.

4. The medical instrument (1) according to claim 3, wherein the narrow band light is light of at least two narrow bands for observing the blood vessel image or the microstructure of the living body mucous membrane.

5. The medical instrument (1) according to claim 1, wherein:

the illumination means is configured to irradiate the normal light; and
the image signal generation means (37, 40, 43, 46) is configured to generate an image signal of the narrow band light by spectral estimation processing based on the returning light of the normal light.

6. The medical instrument (1) according to claim 1, wherein, with respect to a color of an observation object other than the living tissue, the color correction means (48) is configured to change a correction parameter in the color correction in accordance with a luminance level of an image signal of the narrow band light from image pickup means.

7. The medical instrument (1) according to claim 1, wherein, with respect to a color of an observation object other than the living tissue, the color correction means (48) is configured to perform the color correction processing by matrix calculation using a matrix of correction coefficients.

8. The medical instrument (1) according to claim 1, wherein the color correction means (48) is configured to divide hue into ten regions, and perform color correction processing based on the ten hue regions.

9. The medical instrument (1) according to claim 1, wherein the medical instrument (1) is a processor for an endoscope that is configured to process an endoscopic image.

10. The medical instrument (1) according to claim 1, wherein the medical instrument (1) is a capsule endoscope.

**Patentansprüche**

1. Medizinisches Instrument (1), umfassend:

Bildsignal-Erzeugungsmittel (37, 40, 43, 46), die zum Erzeugen eines Bildsignals von normalem Licht und eines Bildsignals von Schmalbandlicht zum Beobachten eines Blutgefäßbildes oder einer Mikrostruktur einer Schleimhaut eines lebenden Körpers auf der Basis von Rückgabelicht von Licht, mit dem lebendes Gewebe durch Beleuchtungsmittel bestrahlt wird, ausgebildet sind;
Farbunterscheidungsmittel (47), die zum zum Identifizieren eines Farbtons für jeweilige Pixel entsprechend einem Leuchtdichtenniveau eines Bildsignals des Schmalbandlichts und Unterscheiden, ob die jeweiligen Pixel einem anderen Beobachtungsobjekt als dem lebenden Gewebe entsprechen oder nicht, ausgebildet sind; und

Farbkorrekturmittel (48), die, in einem Beobachtungsmodus, der das Schmalbandlicht verwendet, auf der Basis des Farbunterscheidungsmittels (47), wenn ein anderes Beobachtungsobjekt als das lebende Gewebe als ein roter Farbton unterschieden wird, zum Durchführen einer Farbkorrektur zu einem gelben Farbton, und wenn ein anderes Beobachtungsobjekt als das lebende Gewebe als ein grüner Farbton oder ein blaugrüner Farbton unterschieden wird, zum Durchführen einer Farbkorrektur zu einem blauen Farbton ausgebildet sind.

2. Medizinisches Instrument (1) nach Anspruch 1, wobei ein Leuchtdichtenniveau eines Bildsignals des Schmalbandlichts ein Leuchtdichtenniveau eines RGB-Signals ist.

3. Medizinisches Instrument (1) nach Anspruch 1, wobei:

das Beleuchtungsmittel zum schaltbaren Ausstrahlen des normalen Lichts und des Schmalbandlichts ausgebildet ist; und
das Bildsignal-Erzeugungsmittel (37, 40, 43, 46) zum Erzeugen eines Bildsignals des Schmalbandlichts, mit dem das lebende Gewebe vom Beleuchtungsmittel bestrahlt wird, ausgebildet ist.

4. Medizinisches Instrument (1) nach Anspruch 3, wobei das Schmalbandlicht Licht von wenigstens zwei Schmalbändern zum Beobachten des Blutgefäßbildes oder der Mikrostruktur der Schleimhaut eines lebenden Körpers ist.

5. Medizinisches Instrument (1) nach Anspruch 1, wobei:

das Beleuchtungsmittel zum Ausstrahlen des normalen Lichts ausgebildet ist; und
das Bildsignal-Erzeugungsmittel (37, 40, 43, 46) zum Erzeugen eines Bildsignals des Schmalbandlichts durch Spektralschätzungsverarbeitung auf der Basis des Rückgabelichts des normalen Lichts ausgebildet ist.

6. Medizinisches Instrument (1) nach Anspruch 1, wobei, in Bezug auf eine Farbe eines anderen Beobachtungsobjekts als das lebende Gewebe, das Farbkorrekturmittel (48) zum Ändern eines Korrekturparameters in der Farbkorrektur entsprechend einem Leuchtdichtenniveau eines Bildsignals des Schmalbandlichts vom Bildaufnahmemittel ausgebildet ist.

7. Medizinisches Instrument (1) nach Anspruch 1, wobei, in Bezug auf eine Farbe eines anderen Beobachtungsobjekts als das lebende Gewebe, das Farbkorrekturmittel (48) zum Durchführen der Farbkorrekturverarbeitung durch Matrixberechnung unter Verwendung einer Matrix von Korrekturkoeffizienten ausgebildet ist.

8. Medizinisches Instrument (1) nach Anspruch 1, wobei das Farbkorrekturmittel (48) zum Unterteilen von Farbton in zehn Bereiche und Durchführen der Farbkorrekturverarbeitung auf der Basis der zehn Farbtonbereiche ausgebildet ist.

9. Medizinisches Instrument (1) nach Anspruch 1, wobei das medizinische Instrument (1) ein Prozessor für ein Endoskop ist, das zum Verarbeiten eines endoskopischen Bildes ausgebildet ist.

10. Medizinisches Instrument (1) nach Anspruch 1, wobei das medizinische Instrument (1) ein Kapselendoskop ist.

**Revendications**

1. Instrument médical (1), comprenant :

des moyens de génération de signal d'image (37, 40, 43,46) configurés pour générer un signal d'image de lumière normale et un signal d'image de lumière à bande étroite pour observer une image de vaisseau sanguin ou une microstructure d'une membrane muqueuse de corps vivant sur la base d'une lumière de retour de lumière qui est irradiée sur un tissu vivant par des moyens d'éclairage ;
des moyens de discrimination de couleur (47) configurés pour identifier un ton de couleur pour des pixels respectifs selon un niveau de luminance d'un signal d'image de la lumière à bande étroite, et discriminer si les pixels respectifs correspondent ou non à un objet d'observation autre que le tissu vivant ; et
des moyens de correction de couleur (48) configurés pour, dans un mode d'observation qui utilise la lumière à bande étroite, sur la base des moyens de discrimination de couleur (47), si un objet d'observation autre que le tissu vivant est discriminé comme étant un ton de couleur rouge, réaliser une correction de couleur en un ton de couleur jaune et, si un objet d'observation autre que le tissu vivant est discriminé comme étant un ton de couleur vert ou un ton de couleur bleu-vert, réaliser une correction de couleur en un ton de couleur bleu.

2. Instrument médical (1) selon la revendication 1, dans lequel un niveau de luminance d'un signal d'image de la lumière à bande étroite est un niveau de luminance d'un signal RVB.

3. Instrument médical (1) selon la revendication 1, dans lequel :

les moyens d'éclairage sont configurés pour irradier de manière commutable la lumière normale et la lumière à bande étroite ; et
les moyens de génération de signal d'image (37, 40, 43,46) sont configurés pour générer un signal d'image de la lumière à bande étroite qui est irradiée sur le tissu vivant par les moyens d'éclairage.

4. Instrument médical (1) selon la revendication 3, dans lequel la lumière à bande étroite est une lumière d'au moins deux bandes étroites pour observer l'image de vaisseau sanguin ou la microstructure de la membrane muqueuse de corps vivant.

5. Instrument médical (1) selon la revendication 1, dans lequel :

les moyens d'éclairage sont configurés pour irradier la lumière normale ; et
les moyens de génération de signal d'image (37, 40, 43,46) sont configurés pour générer un signal d'image de la lumière à bande étroite par traitement d'estimation spectrale sur la base de la lumière de retour de la lumière normale.

6. Instrument médical (1) selon la revendication 1, dans lequel, en ce qui concerne une couleur d'un objet d'observation autre que le tissu vivant, les moyens de correction de couleur (48) sont configurés pour changer un paramètre de correction dans la correction de couleur selon un niveau de luminance d'un signal d'image de la lumière à bande étroite à partir de moyens de saisie d'image.

7. Instrument médical (1) selon la revendication 1, dans lequel, en ce qui concerne une couleur d'un objet d'observation autre que le tissu vivant, les moyens de correction de couleur (48) sont configurés pour réaliser le traitement de correction de couleur par calcul matriciel à l'aide d'une matrice de coefficients de correction.

8. Instrument médical (1) selon la revendication 1, dans lequel les moyens de correction de couleur (48) sont configurés pour diviser une nuance en dix régions, et réaliser un traitement de correction de couleur sur la base des dix régions de nuance.

9. Instrument médical (1) selon la revendication 1, dans lequel l'instrument médical (1) est un processeur pour un endoscope qui est configuré pour traiter une image endoscopique.

10. Instrument médical (1) selon la revendication 1, dans lequel l'instrument médical (1) est une capsule endoscopique.

# FIG.1

EP 2 692 276 B1

# FIG.2

TRANSMITTANCE

Ba    Ga

400 nm    500 nm    600 nm    700 nm    WAVELENGTH (nm)

# FIG.3

TBL

| HUE REGION | RGB LUMINANCE LEVEL RELATIONSHIP |
|---|---|
| (1A) | ((2*B-G) > R) & (R>=B>=G) |
| (1B) | ((2*B-G) <= R) & (R>=B>=G) |
| (2A) | ((2*G-B) <= R) & (R>=G>=B) |
| (2B) | ((2*G-B) > R) & (R>=G>=B) |
| (3) | G>=R>=B |
| (4A) | ((2*B-R) <= G) & (G>=B>=R) |
| (4B) | ((2*B-R) > G) & (G>=B>=R) |
| (5A) | ((2*G-R) > B) & (B>=G>=R) |
| (5B) | ((2*G-R) <= B) & (B>=G>=R) |
| (6) | B>=R>=G |

# FIG.4

# FIG.5

# FIG.6

# FIG.7

# FIG.8

# FIG.9

# FIG.10

# FIG.11

**EP 2 692 276 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004008230 A
- JP 2003079568 A
- JP 2007125245 A
- US 2003001104 A1
- WO 2010044432 A
- JP 2003093336 A
- JP 2011207719 A